Europäisches Patentamt

European Patent Office

Office européen des brevets

(11) Publication number: **0 469 519 A2**

# EUROPEAN PATENT APPLICATION

(21) Application number: **91112718.1**

(22) Date of filing: **29.07.91**

(51) Int. Cl.5: **G01N 33/66**

(30) Priority: **30.07.90 JP 199267/90**

(43) Date of publication of application:
**05.02.92 Bulletin 92/06**

(84) Designated Contracting States:
**CH DE FR GB LI SE**

(71) Applicant: **Tosoh Corporation**
**4560, Kaisei-cho**
**Shinnanyo-shi, Yamaguchi-ken 746(JP)**

(72) Inventor: **Nakamura, Koji**
**3079-3, Oaza-tonda**
**Shinnanyo-shi, Yamaguchi-ken(JP)**
Inventor: **Nakatani, Shigeru**
**26-15-201, Daijin 1-chome**
**Shinnanyo-shi, Yamaguchi-ken(JP)**

(74) Representative: **Wächtershäuser, Günter, Dr.**
**Tal 29**
**W-8000 München 2(DE)**

(54) **Method for analyzing glycoalbumin.**

(57) A method for analyzing glycoalbumin and non-glycoalbumin by liquid chromatography, which comprises separating a sample containing glycoalbumin and non-glycoalbumin by a separation column into a glycoprotein fraction and a non-glycoprotein fraction, and, without withdrawing the eluates out of the flow system of the chromatography, mixing and reacting the respective eluates with a reagent capable of specifically reacting only with albumin to detect the glycoalbumin and the non-glycoalbumin, respectively.

EP 0 469 519 A2

The present invention relates to a method for analyzing glycoalbumin and non-glycoalbumin in a sample by means of liquid chromatography, which is useful particularly as a diagnostic method for diabetes.

It is known that hyperglycemia at the time of diabetes, undergoes a glucose-addition reaction non-enzymatically with various proteins in the vital body. As an index for the blood sugar level, glycohemoglobin ($HbA_{1C}$) which is a combined product of hemoglobin with glucose, reflects the blood sugar level of the past one or two month before the blood was sampled and thus is suitable for a long term administration of diabetes. Accordingly, the glycohemoglobin has been rapidly applied for clinical use, but it has a drawback that the reactivity to the therapeutic effect for a short period of time is dull and it is indispensable to measure blood sugar simultaneously.

On the other hand, glycoalbumin which is a combined product of albumin in blood with glucose, reflects the blood sugar level of from one to two weeks ago and thus is expected to be important as an index for the control of the blood sugar level for a short period of time.

Heretofore, as a method for separating and detecting glycoalbumin, there is a method disclosed in Japanese Unexamined Patent Publication No. 226999/1987. This is a method in which albumin is separated from a sample in the first column, and it is sent to a second column without being withdrawn out of the flow system of chromatography, and in the second column, the albumin is separated into glycoalbumin and non-glycoalbumin. Further, Japanese Unexamined Patent Publication No. 262469/1989 discloses a similar method in which the measuring time has been shortened. Namely, this is a method wherein a plurality of second columns are used to shorten the time for the analysis.

In such a conventional method, a plurality of columns are used, whereby it takes a long time for the analysis, and the analytical cycle tends to be long. Therefore, such a method is not suitable for use for a clinical inspection.

The present inventors have conducted extensive researches to overcome the above problems. As a result, they have found a method for analyzing glycoalbumin which is excellent in the operational speed, the operability and the reproducibility and which can readily be automated. The present invention has been accomplished on the basis of this discovery. Namely, the present invention provides a method for analyzing glycoalbumin and non-glycoalbumin by liquid chromatography, which comprises separating a sample containing glycoalbumin and non-glycoalbumin by a separation column into a glycoprotein fraction and a non-glycoprotein fraction, and, without withdrawing the eluates out of the flow system of the chromatography, mixing and reacting the respective eluates with a reagent capable of specifically reacting only with alubumin to detect the glycoalbumin and the non-glycoalbumin, respectively.

Now, the present invention will be described in detail with reference to the preferred embodiments.

In the accompanying drawings:

Figure 1 is a diagrammatical view showing one embodiment of the apparatus useful for carrying out the method of the present invention.

Figure 2 shows a time program of the mobile phase.

Figure 3 is a chromatogram of non-glycoalbumin and glycoalbumin separated from a blood serum of a healthy person in accordance with the method of the present invention.

In the present invention, glycoalbumin means albumin having glucose covalently bonded thereto; non-glycoalbumin means albumin having no glucose bonded thereto; and albumin means both the glycoalbumin and the non-glycoalbumin.

In the present invention, the sample may be the one which contains either the glycoalbumin or the non-glycoalbumin, or both of them. Further, it may be the one having the glycoalbumin and the non-glycoalbumin separated from other protein components by pretreatment, or the one which contains other glycoprotein component and non-glycoprotein component, such as glyco-IgG and non-glyco-IgG. For example, it may be a body fluid such as blood or urine.

The separation column to be used in the present invention may be a column packed with a gel having dihydroxyboroinc acid. However, there is no particular restriction, so long as it is capable of separating a non-glycoprotein including non-glycoalbumin and a glycoprotein including glycoalbumin. For example, it may be an ion exchange packing material of e.g. carboxymethyl type or sulfopropyl type.

As a carrier for the fixed phase, cellulose, agarose, silica, polyvinyl alcohol or a polyhydroxymethacrylate, which is commonly used in liquid chromatography, may be mentioned. A carrier having excellent mechanical strength and chemical stability is particularly preferred.

As the eluent to separate the glycoalbumin and the non-glycoalbumin by means of the separation column of the present invention, it is preferred to use two types of mobile phase (eluent A and eluent B), so that separation can be conducted by stepwise switching from eluent A to eluent B.

For example, as eluent A, it is preferred to employ an eluent of pH 7.0 to 9.0 containing no substance having 1,2-cis diol and containing from 20 to 500 mM of a buffer solution. As the buffer solution, it is preferred to employ the one having a buffering effect of pH 7.0 to 9.0. Particularly, ammonium acetate or HEPES (N-2-hydroxyethylpiperadine-N'-2-ethanesulfonic acid) may be mentioned. Further, eluent A may contain a salt other than the buffer solution. It is particularly preferred to employ a salt of a bivalent metal ion such as magnesium chloride, since it is thereby possible to strengthen the bond between the glucose chain and the dihydroxyboronyl group. The concentration of the salt of a bivalent metal ion is preferably from 10 to 100 mM.

On the other hand, eluent B may be an eluent of pH 7.0 to 9.0 containing from 20 to 500 mM of a buffer solution so long as it contains a substance having 1,2-cis diol. As the buffer solution, it is preferred to use the same buffer solution as used for eluent A, but other buffer solution may be employed. As the substance having 1,2-cis diol, a saccharide such as sorbitol or mannitol may be mentioned, and its concentration is preferably from 50 to 500 mM.

The reagent capable of specifically reacting only with albumin to be used in the present invention, may be a coloring matter such as Methyl Orange, bromosulfophthalein, Phenol Red, Bromophenol Blue, Bromothymol Blue, Bromocresol Green and Bromocresol Purple, or an anti-human albumin antibody. Among them, Bromocresol Green, Bromocresol Purple and an anti-human albumin antibody are particularly preferred, since they are not substantially influenced by other components in blood serum.

The reaction product of albumin with the reagent can be detected by absorptiometry.

When the detection is conducted by absorptiometry by means of the coloring matter or the anti-human albumin antibody, the wavelength for the measurement is from 600 to 650 nm, or from 300 to 900 nm, respectively.

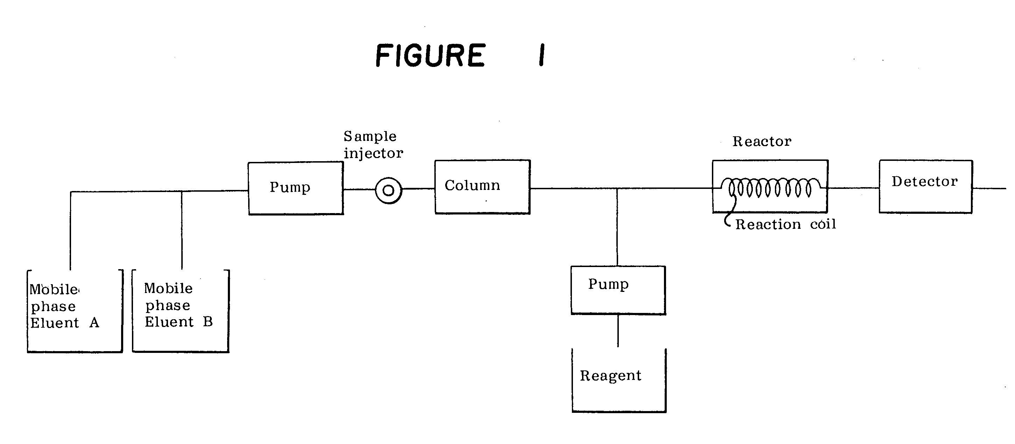

One embodiment of the apparatus for carrying out the method of the present invention is shown in Figure 1.

The method of the present invention for analyzing glycoalbumin and non-glycoalbumin is characterized in that in liquid chromatography, a glycoprotein fraction containing glycoalbumin and a non-glycoprotein fraction containing non-glycoalbumin in the sample are separated by a separation column, and without withdrawing them out of the flow system of the chromatography, they are mixed with the reagent capable of specifically reacting only with albumin to detect the glycoalbumin and the non-glycoalbumin, respectively.

According to the method of the present invention, the analysis can readily and simply be conducted even in a usual clinical inspection room, and the analysis can readily be automated. Thus, the present invention contributes significantly to the progress in the analysis of glycoalbumin. Now, the present invention will be described with reference to an Example. However, it should be understood that the present invention is by no means restricted to such a specific Example.

EXAMPLE

As the column, a stainless steel column having an inner diameter of 4.6 mm and a length of 1 cm packed with TSKgel Boronate-5PW, manufactured by TOSOH CORPORATION, was used. The detailed conditions for chromatography are as shown below.

Sample:
Human blood serum (5 $\mu\ell$)
Mobile phase:
eluent A: 50 mM ammonium acetate/20 mM Mgc$\ell_2$/0.05% NaN$_3$ (pH 8)
eluent B: 50 mM ammonium acetate/20 mM Mgc$\ell_2$/0.2 M sorbitol/0.05% NaN$_3$ (pH 8)
Pump:
CCPM, manufactured by TOSOH CORPORATION
Flow rate of the mobile phase:
0.8 m$\ell$/min
Separation column temperature:
25°C
Reagent:
0.5 M succinic acid/0.2 mM Bromocresol Purple/0.05% Brij-35 (pH 5.5)
Flow rate of the reagent:
1.5 m$\ell$/min
Reaction coil:
0.6 mm x 3 m
Reactor:
RE8010, manufactured by TOSOH CORPORATION
Reactor temperature:
37°C
Detector:
UV8010, manufactured by TOSOH CORPORATION
Wavelength of the detector:
620 nm

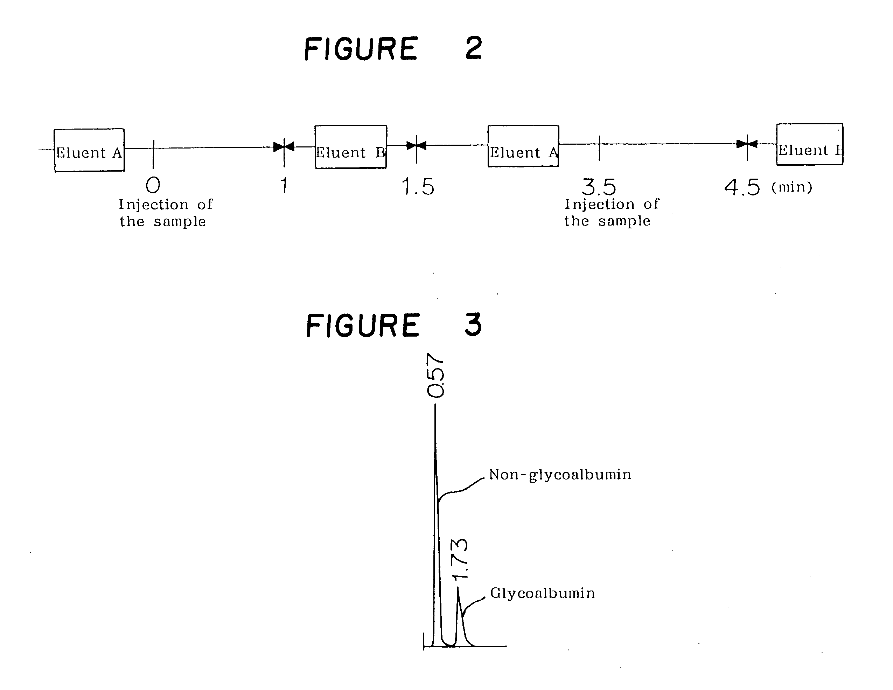

The mobile phase was switched in accordance with the time program as shown in Figure 2.

The chromatogram obtained under the above conditions is shown in Figure 3. It is apparent that the non-glycoalbumin and the glycoalbumin can be analyzed in a short period of time (i.e. 3.5 minutes cycle).

**Claims**

1. A method for analyzing glycoalbumin and non-glycoalbumin by liquid chromatography, which comprises separating a sample containing glycoalbumin and non-glycoalbumin by a separation column into a glycoprotein fraction and a non-glycoprotein fraction, and, without withdrawing the eluates out of the flow system of the chromatography, mixing and reacting the respective eluates with a reagent capable of specifically reacting only with albumin to detect the glycoalbumin and the non-glycoalbumin, respectively.

2. The method according to Claim 1, wherein the sample is urine or blood.

3. The method according to Claim 1, wherein the separation column is a column packed with a gel having dihydroxyboric acid, or an ion exchange packing material.

4. The method according to Claim 1, wherein the separation is conducted by stepwise switching from eluent A of pH 7.0 to 9.0 containing no substance having 1,2-cis diol to eluent B of pH 7.0 to 9.0 containing from 50 to 500 mM of a substance having 1,2-cis diol.

5. The method according to Claim 4, wherein eluent A contains from 10 to 100 mM of a salt of a bivalent metal ion.

6. The method according to Claim 4, wherein the substance having 1,2-cis diol is a saccharide.

7. The method according to Claim 6, wherein the saccharide is sorbitol or mannitol.

8. The method according to Claim 1, wherein the reagent capable of specifically reacting only with albumin is a coloring matter selected from the group consisting of Methyl Orange, bromosulfophthalein, Phenol Red, Bromophenol Blue, Bromothymol Blue, Bromocresol Green and Bromocresol Purple, or an anti-human albumin antibody.

9. The method according to Claim 1, wherein the reagent capable of specifically reacting only with albumin is Bromocresol Blue, Bromocresol Purple or an anti-human albumin antibody.

# FIGURE 1

Sample injector · Reactor · Detector · Pump · Column · Mobile phase Eluent A · Mobile phase Eluent B · Pump · Reagent · Reaction coil

EP 0 469 519 A2

# FIGURE 2

| Eluent A | | Eluent B | | Eluent A | | Eluent E |
|---|---|---|---|---|---|---|

0 — Injection of the sample

1

1.5

3.5 — Injection of the sample

4.5 (min)

# FIGURE 3

0.57 — Non-glycoalbumin

1.73 — Glycoalbumin

EP 0 469 519 A2